# EUROPEAN PATENT APPLICATION

(11) **EP 2 319 570 A1**
(43) Date of publication of application: **11.05.2011**
(21) Application number: 10181509.0
(22) Date of filing: 22.11.2001
(51) Int. Cl.: A61M 16/06, A62B 18/02, A62B 18/10

(54) **Respiratory mask and gas washout vent**

(62) Divisional of application: 08153449.7
(71) Applicant: ResMed Limited, North Ryde, New South Wales 2113 (AU)
(72) Inventor: Drew, Joanna Elizabeth, Balgowlah New South Wales 2093 (AU); Virr, Alexander, Balmain New South Wales 2041 (AU); Crumblin, Geoffrey, Baulkham Hills New South Wales 2153 (AU)
(74) Representative: Vossius & Partner

(57) **Abstract**

A respiratory mask (10), particularly of the type used in CPAP treatment, has a gas washout vent (26,28). In one embodiment the vent (26,28) is made of a thin air-permeable membrane (28). Generally, the membrane (28) is thinner than about 0.5 mm. The membrane (28) can be made of a hydrophobic material such as polytetrafluoroethylene (PTFE). The membrane (28) can also be fabricated from expanded PTFE. In an alternative embodiment, the vent assembly includes a vent constructed from stainless steel.

## Description

The present invention relates to a respiratory mask and to a vent for a respiratory mask.

The application of Continuous Positive Airway Pressure (CPAP) via a nasal mask is a common ameliorative treatment for sleep disordered breathing (SDB) including obstructive sleep apnoea (OSA), as described for example in the applicant's US-A-4944310. In CPAP treatment for OSA, air or other breathable gas is supplied to the entrance of a patient's airways at a pressure elevated above atmospheric pressure, typically in the range 3-20 cm H₂0 as measured in the patient interface. It is also known for the level of treatment pressure to vary during a period of treatment in accordance with patient need, that form of CPAP being known as automatically adjusting nasal CPAP treatment as described for example in the applicant's US-A-5245995.

Non-invasive positive pressure ventilation (NIPPV) is another form of treatment for breathing disorders including sleep disorder breathing. In a basic form, NIPPV involves a relatively high pressure of gas being provided in the patient interface during the inspiratory phase of respiration and a relatively low pressure or atmospheric pressure being provided in the patient interface during the expiratory phase of respiration. In other NIPPV modes, the pressure can be made to vary in a complex manner throughout the respiratory cycle. For example, the pressure at the patient interface during inspiration or expiration can be varied through the period of treatment as disclosed in the applicant's WO-A-98/12965 and WO-A-99/61088.

In this specification, any reference to CPAP treatment is to be understood as embracing all of the above-described forms of ventilatory treatment or assistance.

Typically, the patient interface for CPAP treatment consists of a nasal mask. The nasal mask is generally defined by a mask shell which forms an inner cavity defined by its interior surface, mask cushion and the user's face, and a gas inlet which may or may not include a separate component such as a swivel elbow. Alternatively, a nose-mouth mask or full-face mask or nasal prongs or nasal pillows can be used. In this specification, any reference to a mask is to be understood as incorporating a reference to a nasal mask, nose-mouth mask, full face mask, nasal prongs or nasal pillows unless otherwise specifically indicated. The mask incorporates, or has in close proximity, a gas washout vent for venting exhaled gases to atmosphere. The gas washout vent (the vent) is sometimes referred to as a CO₂ washout vent.

It is important that the apparatus is quiet and comfortable to encourage patient compliance with therapy. The exhausting to atmosphere of exhaled gas through the vent creates noise. As CPAP and NIPPV treatments are normally administered while the patient is sleeping, minimisation or such noise is desirable for both the comfort of the patient and any bed partner.

From a clinical perspective it is desirable for a mask and vent combination to maximise both the elimination of exhaled CO₂ through the vent and also the inhalation of the supplied breathable gas. In this way, retention of exhaled CO₂ within the mask, which is "re-breathed" by the wearer, is minimised. Generally, by locating the vent in the mask shell, CO₂ washout will be superior to locating the same vent between the mask shell and the breathable gas supply conduit.

It is desirable to minimise the weight of the vent assembly for greater patient comfort.

Systems for the delivery of nasal CPAP treatment often incorporate in-line humidifiers to minimise drying of the nasal mucosa and increase patient comfort. Accordingly, it is also desirable that a vent not block when used with humidified gas. It is also desirable that a vent be easily cleaned or economically disposable.

A number of vent configurations are known. One approach to vent configuration is to create within the mask shell one or more openings that allow for the flow of exhaust gas from the inner cavity to atmosphere. The exhaust flow may be directed through the incorporation of an additional pipe extending out from the opening located on the mask shell outer surface.

The applicant's nasal mask system known by the name ResMed Modular Mask System incorporates an outlet vent located in the swivel elbow connected to the mask shell. The ports defining the vent have the same cross-sectional thickness and are formed from the same polycarbonate material that is used to form the swivel elbow and mask shell frame.

The whisper swivel, manufactured by Respironics, Inc., provides three slots on the circumference of a generally cylindrical attachment piece. In use, the attachment piece is to be interposed between the mask shell and the gas conduit. The attachment piece is made of the same material and thickness as is used to make the mask shell.

EP-A-0697225 discloses a vent formed from a porous sintered material.

A known vent, manufactured by Gottleib Meinmann Geräte Für Medizin Und Arbeitsschutz GmbH and Co., comprises a generally cylindrical insert to be interposed in use between the mask shell and the gas conduit. The insert includes a window which is covered with a porous sintered material of approximately 3-4mm thickness.

Another type of vent intended to be inserted between the mask shell and the breathable gas supply conduit is the E-Vent N by Draeger Medizintechnik GmbH (the Draeger vent). The Draeger vent comprises a stack of 21 annular disks, which have slots in their adjacent surfaces for gas to flow therethrough. Each slot has a length of 5 to 7mm as measured along the path from the interior of the vent to atmosphere.

The applicant produces a respiratory mask known as the MIRAGE^{®} nasal mask system and the MIRAGE^{®} full-face mask (the MIRAGE mask). The MIRAGE^{®} mask has a crescent shaped opening in the mask shell in which is located a complementary shaped crescent elastomeric insert with six holes therein which constitutes the vent. The elastomeric inset has a cross-sectional thickness of 3 to 4mm. The vent of the type used in the MIRAGE^{®} is described in WG-A-98/34665 and Australian Patent No. 712236.

It is an object of the present invention to provide an alternative form of vent that is suitable for use in a respiratory mask.

According to a first aspect of the present invention, there is provided a respiratory mask comprising: a mask frame; a cushion provided to the mask frame; a breathable gas inlet to conduct gas through the mask frame to a breathing cavity formed between the mask frame and a user's face when the mask is in use; and a gas washout vent; the vent including an air permeable membrane formed and constructed to have a plurality of holes or pores **characterised in that** the membrane is thinner than 0.5 mm.

According to a second aspect of the present invention, there is provided a gas washout vent for use in a respiratory mask, the vent comprising an air-permeable membrane.

The preferred embodiment of the present invention provides a vent assembly suitable for use with a mask used in CPAP treatment wherein the vent assembly is a thin air-permeable membrane.

The membrane may be thinner than the mask frame.

The membrane may be thinner than about 0.5mm.

The membrane may have an approximate thickness of 0.05mm.

The membrane may be constructed from a hydrophobic material. A suitable material is polytetrafluoroethylene (PTFE).

The membrane may be constructed from expanded PTFE. The expanded PTFE membrane may be mounted on a polypropylene scrim.

The pores of the membrane may have a reference pore size of 10 to 15 microns.

The membrane may be constructed from stainless steel.

The membrane of the vent may have a superficial cross-sectional area of approximately 500mm².

The vent assembly may comprise a membrane attached to a vent frame, the vent assembly forming an insert which can be removably attached to a mask fame.

In an embodiment, there may be provided a respiratory mask for communicating breathable gas to the entrance of a wearer's airways, the mask including (i) mask shell, (ii) a gas inlet and (iii) an opening into which an insert constructed from a thin air permeable membrane with a corresponding shape may be placed. The opening may be positioned in the mask shell or in the gas inlet.

In one form, the mask includes a mask shell with an integrally formed gas inlet and the opening is provided in the mask shell remote the inlet. In another form, the mask includes a mask shell with an integrally formed gas inlet and the opening is provided in the gas inlet. In yet another form, the mask includes a mask shell with a separately formed gas inlet attached thereto and the opening is provided in the mask shell remote the inlet. In still yet another form, the mask includes a mask shell with a separately formed gas inlet attached thereto and the opening is provided in the gas inlet.

In an embodiment, the present invention also provides a respiratory mask arrangement for communicating breathable gas to the entrance of a wearer's airways, the mask arrangement including a vent assembly comprising an opening with a thin air permeable membrane extending across an opening.

In an embodiment, the present invention also provides an apparatus for delivering CPAP which apparatus includes a mask arrangement for communicating breathable gas to the entrance of a wearer's airways, the mask arrangement including a gas washout vent assembly comprising an opening with a thin air permeable membrane extending across said opening.

Further preferred aspects of the invention are exemplified in the following:
1. A respiratory mask, the respiratory mask comprising:
   a mask shell that can be fitted over a user's nose;
   a breathable gas inlet that can conduct gas through the shell to a breathing cavity formed between the shell and a user's face when the mask is in use; and,
   a gas washout vent comprising an air-permeable membrane for allowing gas to exit from the breathing cavity,
   wherein the membrane is thinner than the mask frame.
2. A respiratory mask according to aspect 1, wherein the gas washout vent is located in the mask shell.
3. A respiratory mask according to aspect 1, wherein the gas washout vent is located in the gas inlet.
4. A respiratory mask according to any one of aspects 1 to 3, wherein the air-permeable membrane comprises a stainless steel sheet having holes therein.
5. A respiratory mask according to any one of aspects 1 to 4, wherein the total area of the holes constitutes substantially 5% of the area of the membrane.
6. A respiratory mask according to any one of aspects 1 to 5, wherein the membrane is substantially 0.45mm thick.
7. A respiratory mask according to any one of aspects 1 to 6, wherein the membrane has an area of approximately 322mm².
8. A respiratory mask according to any one of aspects 1 to 7, wherein the air-permeable membrane is made from a hydrophobic material.
9. A respiratory mask according to any one of aspects 1 to 7, wherein the air-permeable membrane comprises expanded polytetrafluoroethylene.
10. A respiratory mask according to aspect 9, wherein the air-permeable membrane comprises a GORE-TEX® membrane provided on a scrim.
11. A respiratory mask according to aspect 10, wherein the scrim is made of polypropylene.
12. A respiratory mask according to any one of aspects 1 to 11, wherein the membrane has an approximate pore size in the range of 10 to 15 microns.
13. A respiratory mask according to any one of aspects 1 to 12, wherein the membrane has an area of approximately 480mm².
14. A respiratory mask according to any one of aspects to 13, wherein the membrane has thickness of approximately 0.05mm
15. A respiratory mask according to any one of aspects to 14, comprising a cushion positioned in proximity to an edge portion of the shell to aid in fitting the mask shell to a user's face.
16. A respiratory mask according to any one of aspects 1 to 15, wherein the air-permeable membrane is less than about 0.5mm thick.
17. A respiratory mask according to any one of aspects 1 to 16, wherein the air-permeable membrane has a plurality of holes.
18. A respiratory mask according to any one of aspects 1 to 17, wherein the vent is a vent assembly comprising a membrane attached to a vent frame, the vent assembly forming an insert which can be removably attached to the mask shell.
19. A respiratory mask according to aspect 17 or 18, wherein the holes are tapered.
20. A respiratory mask according to any one of aspects 17 to 19, wherein the holes are tapered so that a larger end of the vent's openings is located on the atmosphere side.
21. A respiratory mask according to any one of aspects to 19, wherein the holes are tapered so that a smaller end of the vent's openings is located on the atmosphere side.
22. A respiratory mask according to any one of aspects to 21, wherein the holes are tapered in a gradual or stepped manner.
23. A respiratory mask according to any of aspects to 22, wherein the holes are regularly shaped.
24. A respiratory mask according to any of aspects 1 to 23, wherein the membrane has a bubble point of at least about 0,02 bar.
25. A respiratory mask according to any of aspects 1 to 24, wherein the airflow capacity of the membrane (28) is about 0.37LPM/cm².
26. A respiratory mask according to an y one of aspects 1 to 25, wherein the membrane includes holes and in that a total open area of the holes is approximately 1-25% of a superficial surface area of the membrane (28).
27. A respiratory mask according to any one of aspects 1 to 26, wherein the holes are approximately 0,1 mm, in diameter.
28. A respiratory mask according to any one of aspects 1 to 26, wherein the holes are less than 0,2 mm in diameter.
29. A respiratory mask according to any one of aspects 1 to 28, wherein the membrane has a plurality of holes, preferably more than 10 and more preferably more than 20 holes.
30. A respiratory mask according to any one of aspects 17 to 29, wherein said plurality of holes is arranged in a plurality of rows and/or a plurality of lines.
31. A respiratory mask according to of aspect 30, wherein each of said plurality of rows and/or a plurality of lines comprises at least 3 of said holes.

Embodiments of the present invention will now be described by way of example with reference to the accompanying drawings, in which:
Fig. 1 is a perspective view of a first example of a respiratory mask according to the present invention;
Fig. 2 is a perspective view of a second example of a respiratory mask according to the present invention;
Fig. 3 is a perspective view of a third example of a respiratory mask according to the present invention;
Fig. 4 is a partial cross-sectional view of an example of a vent assembly according to the present invention;
Fig. 5 is a partial cross-sectional view of an example of a vent assembly according to the present invention;
Fig. 6 is a perspective view of a fourth example of a respiratory mask according to the present invention;
Fig. 7 is a perspective view of an example of a full-face mask according to the present invention;
Fig. 8 is an enlarged detailed view of an example of an insert suitable for use with the masks shown in Fig. 6 and Fig 7; and,
Fig. 9 is a perspective view of another example of a respiratory mask according to the present invention where the thin air-permeable membrane is located in a cylindrical position on a tube suitable for attachment to the mask elbow.

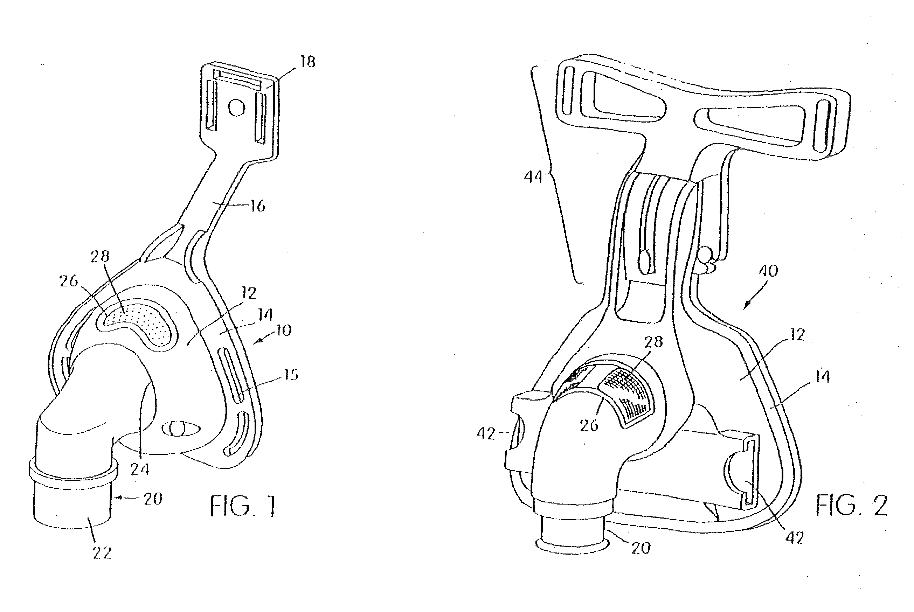

Fig. 1 shows an example of a nasal respiratory mask 10 according to the present invention. The mask 10 includes a rigid plastic mask shell 12 which has a peripheral flange 14 for mounting of a cushion (not shown) to the shell 12. The cushion abuts the wearer's face in use and is well known in the art. The flange 14 includes slots 15 for the connection of mask restraining straps (not shown) that extend around the head of the wearer in use to maintain the mask 10 adjacent to the wearer's face. The straps are also known in the art. The shell 12 also includes an arm 16 which terminates in a fitting 18 that is adapted to connect to a forehead support (not shown), which is also known in the art.

The mask shell 12 includes a breathable gas inlet 20 which is rotatably mounted to the shell 12. The inlet 20 has a first end 22 which is adapted for connection with a breathable gas supply conduit (not shown) and a second end 24 which is adapted to connect to and communicate the supplied gas to the interior of the shell 12 for subsequent communication with the wearer's airways.

The mask 10 includes a gas washout vent constituted by an opening 26 in the shell 12 across which extends a thin air-permeable membrane 28.

In the Fig. 1 embodiment, the thin air-permeable membrane 28 is a stainless steel sheet approximately 0.45mm thick having holes approximately 0.1mm in diameter. The total open area is approximately 5% of the total superficial surface area of the sheet, which may for example be approximately 322mm². The holes are desirably laser cut or flame cut through the stainless steel.

More generally, the holes preferably have a diameter of less than 0.2mm, and preferably provide a total open area of approximately 1% to 25% of the superficial surface area of the steel. The holes may be tapered (in a gradual or stepped manner) through their internal bore. In use, if the smaller end of the vent's openings are located on the atmosphere side, the opportunity for blockage occurring by the insertion of paniculate matter will be minimised. Alternatively, the larger end of the vent's openings may be located on the atmosphere side, which may make the vent quieter.

Fig. 2 shows a second example of a nasal respiratory mask 40 according to the present invention. Like reference numerals to those used in describing the first embodiment will be used to denote like features in respect of the second embodiment. Accordingly, the mask 40 has a shell 12 with a gas inlet 20. Instead of the slots 15 of the first embodiment, the mask shell 12 of this embodiment includes openings 42 which are adapted to snap engage with connection fittings (not shown) provided on the end of mask restraining straps (not shown). Further, instead of the arm 16 and fitting 18, the mask 40 includes an adjustable forehead support mechanism 44. The mask 40 also includes a vent constituted by an opening 26 formed in the gas inlet 20 across which extends a thin air-permeable membrane 28.

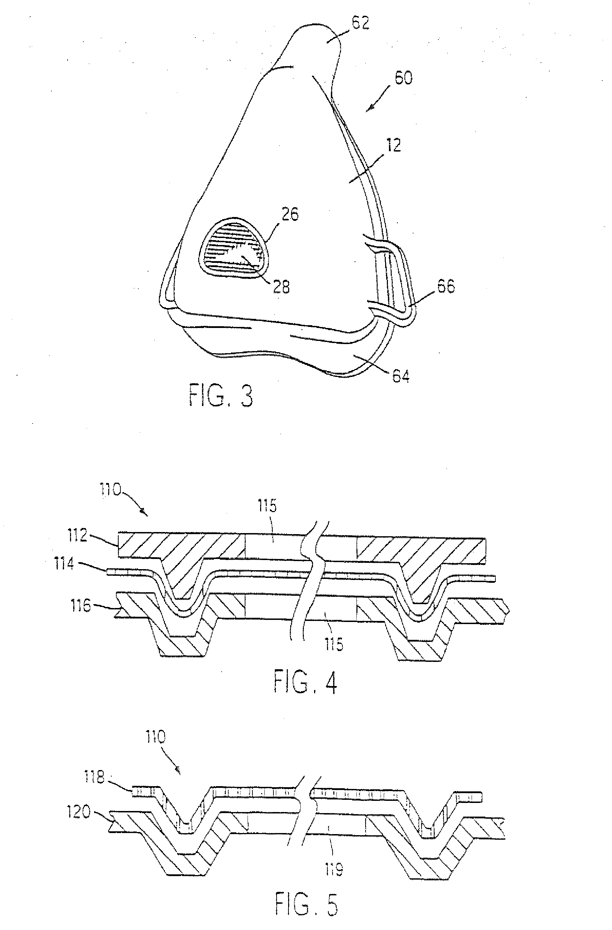

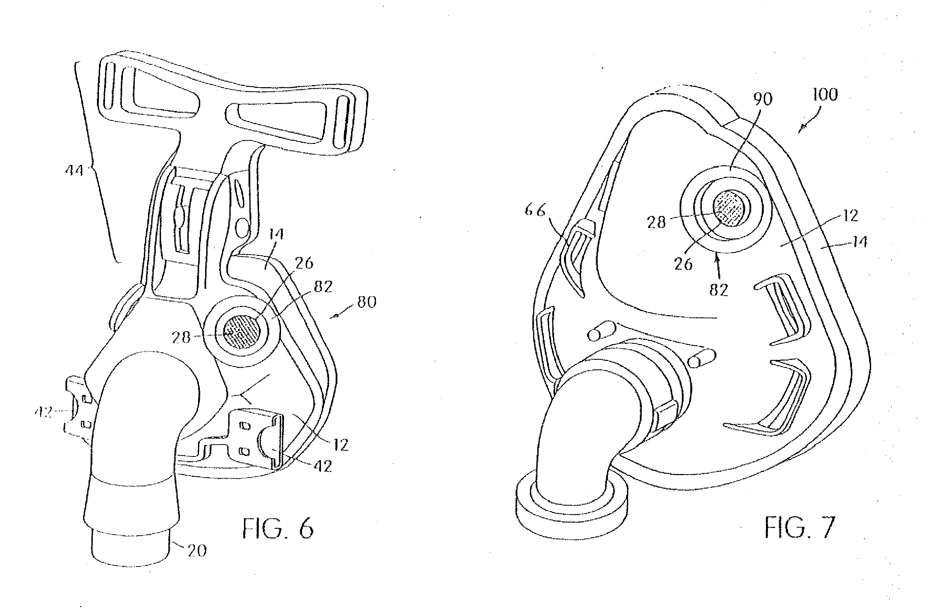

Fig. 3 shows a third example of a mask 60 according to the present invention. Although this particular embodiment is directed to a nasal mask, it should be noted that various vent arrangements can be used with various mask arrangements. Once again, like reference numerals to those used in describing features of the first embodiment will be used to denote like features in respect of the third embodiment. The mask 60 includes a mask shell 12 with an integrally formed fixed gas inlet 62. A cushion 64 is attached to the peripheral flange of the shell 12. The shell 12 also includes slotted extensions 66 for connecting headgear (not shown) to the mask 60. The mask 60 includes an opening 26 across which is extended a thin air-permeable membrane 28 of identical construction to the ePFTE membrane discussed below in relation to the mask 40 shown in Fig. 6.

Fig. 4 shows a cross-section of an example of a vent assembly 110. A membrane 114 is interposed between an outer element 112 and an inner element 116. This arrangement provides for a simple assembly. Corresponding openings 115 in the outer element 112 and inner element 116 allow for the passage of air through the membrane 114. The inner element 116 may form part of the mask frame or of a separate insert to be positioned in an opening in the mask frame.

Fig. 5 shows a cross-section of another example of a vent assembly 110. A stainless steel membrane insert 118 is positioned over an inner element 120. An opening 119 in the inner element 120 allows for the passage of air through the membrane 118. The inner element 119 may form part of the mask frame or of a separate insert to be positioned in an opening in the mask frame.

Fig. 6. shows a fourth example of a nasal respiratory mask 80 according to the present invention. The mask 80 is similar to the second embodiment of the mask 40 shown in Fig. 2 and like reference numerals have been used to indicate like features with respect to the second embodiment. In the mask 40 of Fig. 2, the vent is provided in the gas inlet 20, whereas in the mask 80 the vent is provided in the shell 12. More particularly, the mask 80 includes two cylindrical inserts 82 which have an inner opening 26 across which extends the thin air-permeable membrane 28. The thin air-permeable membrane is made from GORE-TEX^{Ⓗ} product attached to a polypropylene scrim having an area of 481mm². The membrane 28 of this example is preferably constructed from expanded polytetrafluoroethylene (ePTFE). The inventors have identified GORE-TEX^{Ⓡ} ePTFE product manufactured by W.L. Gore & Associates, Inc. of Maryland USA (GORE-TEX^{®} membrane) as being a suitable material for constructing a membrane. In one preferred form, the GORE-TEX^{Ⓡ} membrane has the following characteristics:

| | |
|---|---|
| Membrane material | 100% expanded polytetrafluoroethylene |
| Reference pore size | 10-15 micron |
| Bubble Point | typical minimum individual 0.02 bar |
| Airflow | 0.37LPM/cm² |
| Thickness | 0.05mm |
| Substrate | polypropylene scrim |

Fig. 7 shows an example of a full-face respiratory mask 100 according to the present invention. Once again, like reference numerals to those used in denoting like features with previous embodiments have been used to denote like features in respect of this embodiment. The mask 100 is similar to the mask 80 shown in Fig. 6 in that the vent is provided in inserts 82. However, the mask 100 uses slotted extensions 66 to attach mask restraining straps (not shown), not openings 42.

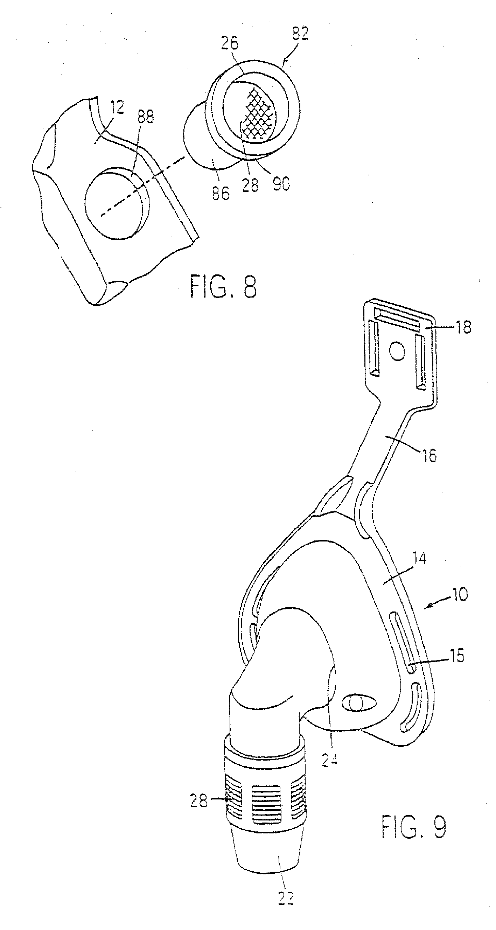

As best seen in Fig. 8, which is a close-up view of the insert shown in Fig. 6, the insert 82 comprises a cylindrical portion 86 sized to be a snug fit into a circular orifice 88 provided in the mask shell 12. The insert 82 is located against the outer surface of the shell 12 by a peripheral flange 90. The inserts 82 may be glued in position.

Fig. 9 shows a further example of the present invention in which an in-line vent assembly is provided. Like numerals are used to indicate like features with previous embodiments. In this embodiment, the in-line vent assembly comprises a generally cylindrical shape vent frame with "windows" or "ports" covered with a membrane as described above.

The thin air-permeable membrane may be attached to the mask by any suitable means. For example, the stainless steel vent describe above may be attached to a polycarbonate mask shell by way of hot glue adhesive or any other suitable adhesive. The durability sought to be achieved will determine the suitable approach for attachment.

In a further embodiment, there is provided a means to indicate the volume of air that has passed through the vent or alternatively the time that the vent assembly has been used. When a sufficient volume of air has passed through the vent assembly, or the assembly has been used for a sufficient time and may have become blocked, the indicator will signal that the vent assembly should be replaced.

For convenience, the thin air-permeable membrane can be provided in an insert which is releasably attachable to the mask shell via a push-fit mechanism, as shown for example in Fig. 8. Preferably, on at least the outer surface of the insert there is provided at least one crosspiece that protects the air-permeable membrane from being damaged as it is located into the receiving orifice of the mask shell. This approach will allow for the easy placement, removal and replacement of a vent insert whilst retaining the other components of the mask. Whilst the insert may be configured to take the form of any requisite shape, preferably the insert has a circular circumferential shape defining a cylindrical insert which has a frictional fit within a corresponding circular orifice in the mask shell or gas inlet.

Formation of the vent through use of an insert configuration facilitates the selection and fitting of a vent to suit a user's requirements. For example, where a low treatment pressure is required, the associated flow will also be relatively small compared with flaw required to achieve a higher treatment pressure. In such circumstances, a relatively large vent area may be adopted to facilitate achievement of the clinically desirable mask CO₂ washout rate. Should a higher treatment pressure be required then the previously selected vent may be exchanged for a vent which is more restrictive to flow. The more restrictive vent allows achievement of the clinically desirable mask CO₂ washout rate whilst avoiding the intensity of noise and exhaust gas jetting that might occur had the previously selected low pressure vent been used with the higher treatment pressure.

Locating the vent in the mask shell results in an improvement in the minimisation of CO₂ retention within the mask compared to locating the vent as an inline mask component.

Embodiments of the present invention have been described with particular reference to the examples illustrated. However, it will be appreciated that variations and modifications may be made to the examples described within the scope of the present invention.

## Claims

1. A respiratory mask, the respiratory mask comprising:
a mask shell that can be fitted over a user's nose;
a breathable gas inlet which can conduct gas through the shell to a breathing cavity formed between the shell and
a user's face when the mask is in use;
the shell including an arm terminating in a fitting adapted to connect to a forehead support and,
a gas washout vent provided in the gas inlet comprising an air-permeable membrane having a plurality of holes for allowing gas to exit from the breathing cavity.

2. A respiratory mask according to any one of the preceding claims, wherein the total area of the holes constitutes substantially 5% of the area of the membrane, and/or wherein the membrane has an area of approximately 322mm², and/or wherein the membrane has an area of approximately 480mm².

3. A respiratory mask according to any one of the preceding claims, wherein the membrane is less than about 0.5mm thick, and/or wherein the membrane is substantially 0.45mm thick, and/or wherein the membrane has thickness of approximately 0.05mm.

4. A respiratory mask according to any one of the preceding claims, wherein the air-permeable membrane is made from a hydrophobic material.

5. A respiratory mask according to any one of the preceding claims, wherein the membrane has an approximate pore size in the range of 10 to 15 microns.

6. A respiratory mask according to any one of the preceding claims, comprising a cushion positioned in proximity to an edge portion of the shell to aid in fitting the mask shell to a user's face.

7. A respiratory mask according to any one of the preceding claims, wherein the holes are tapered so that a larger end of the vent's openings is located on the atmosphere side or so that a smaller end of the vent's openings is located on the atmosphere side, and/or wherein the holes are tapered in a gradual or stepped manner.

8. A respiratory mask according to any of the preceding claims, wherein the holes are regularly shaped.

9. A respiratory mask according to any of the preceding claims, wherein the membrane has a bubble point of at least about 0,02 bar.

10. A respiratory mask according to any of the preceding claims, wherein the airflow capacity of the membrane (28) is about 0.37LPM/cm².

11. A respiratory mask according to any one of the preceding claims, wherein a total open area of the holes is approximately 1-25% of a superficial surface area of the membrane (28).

12. A respiratory mask according to any one of the preceding claims, wherein the membrane is thinner than the mask frame.

13. A respiratory mask according to any one of the preceding claims, wherein the holes are approximately 0,1 mm in diameter, or wherein the holes are less than 0,2 mm in diameter.

14. A respiratory mask according to any one of claims 1 to 27, wherein the membrane has a plurality of holes, preferably more than 10 and more preferably more than 20 holes, and wherein said plurality of holes is preferably arranged in a plurality of rows and/or a plurality of lines, while each of said plurality of rows and/or a plurality of lines preferably comprises at least 3 of said holes.
